# EUROPEAN PATENT SPECIFICATION

(11) **EP 4 281 164 B1**
(45) Date of publication and mention of the grant of the patent: **28.10.2026**
(21) Application number: 22742986.7
(22) Date of filing: 10.01.2022
(51) Int. Cl.: A61M 25/09, A61L 31/02, A61M 25/01, A61L 31/08

(54) **GUIDEWIRE AND METHOD OF USE**
FÜHRUNGSDRAHT UND VERWENDUNGSVERFAHREN
FIL-GUIDE ET SON PROCÉDÉ D'UTILISATION

(30) Priority: 21.01.2021 US 202117154938
(43) Date of publication of application: 29.11.2023
(73) Proprietor: Abbott Cardiovascular Systems Inc., Santa Clara, California 95054 (US)
(72) Inventor: GILL, Puneet Kamal Singh, Anaheim, California 92802 (US); DURCAN, Jonathan P., Temecula, California 92592 (US); VASQUEZ, Matthew, Temecula, California 92592 (US)
(74) Representative: Boult Wade Tennant LLP
(86) International application number: PCT/US2022/011786
(87) International publication number: WO 2022/159297

(56) References cited:
- US-A- 6 142 975
- US-A1- 2002 013 540
- US-A1- 2002 013 540
- US-A1- 2004 030 265
- US-A1- 2005 027 214
- US-A1- 2018 311 477
- US-A1- 2018 311 477

## Description

### BACKGROUND

Conventional guidewires for angioplasty and other vascular procedures usually comprise an elongated core member with one or more tapered sections near the distal end thereof and a flexible body such as a helical coil disposed about the distal portion of the core member. A shapeable member, which may be the distal extremity of the core member or a separate shaping ribbon which is secured to the distal extremity of the core member extends through the flexible body and is secured to a rounded tip at the distal end of the flexible body.

In a typical coronary procedure, a guidewire having a preformed distal tip is percutaneously introduced into a patient's peripheral artery, e.g., the guidewire crosses a lesion to be dilated, then the dilatation catheter having an inflatable balloon on the distal portion thereof is advanced into the patient's coronary anatomy over the previously introduced guidewire until the balloon of the dilatation catheter is properly positioned across the lesion. Once in position across the lesion, the procedure is performed.

A requirement for guidewires is that they have sufficient column strength to be pushed through a patient's vascular system or other body lumen without kinking. However, guidewires must also be flexible enough to avoid damaging the blood vessel or other body lumen through which they are advanced. Efforts have been made to improve both the strength and flexibility of guidewires to make them more suitable for their intended uses, but these two properties are for the most part, diametrically opposed to one another in that an increase in one usually involves a decrease in the other.

Some guidewires have been formed from a superelastic alloy such as a nitinol (nickel-titanium or NiTi) alloy, to achieve both flexibility and strength. When stress is applied to nitinol alloy exhibiting superelastic characteristics at a temperature at or above which the transformation of martensite phase to the austenite phase is complete, the specimen deforms elastically until it reaches a particular stress level where the alloy then undergoes a stress-induced phase transformation from the austenite phase to the martensite phase. As the phase transformation proceeds, the alloy undergoes significant increases in strain but with little or no corresponding increases in stress. The strain increases while the stress remains essentially constant until the transformation of the austenite phase to the martensite phase is complete. Thereafter, further increase in stress are necessary to cause further deformation.

If the load on the specimen is removed before any permanent deformation has occurred, the martensitic phase of the specimen will elastically recover and transform back to the austenite phase. The reduction in stress first causes a decrease in strain. As stress reduction reaches the level at which the martensite phase transforms back into the austenite phase, the stress level in the specimen will remain essentially constant until the transformation back to the austenite phase is complete, i.e., there is significant recovery in strain with only negligible corresponding stress reduction. After the transformation back to austenite is complete, further stress reduction results in elastic strain reduction. This ability to incur significant strain at relatively constant stress upon the application of a load and to recover from the deformation upon the removal of the load is commonly referred to as superelasticity. These properties to a large degree allow a guidewire core of a superelastic material to have both flexibility and strength.

While the properties of the guidewire formed of the superelastic material were very advantageous, it was found that the guidewires and guiding members formed of materials having superelastic characteristics did not have optimum push and torque characteristics.

As is known in the art, many materials used for guidewire construction have desirable mechanical properties, but are difficult to assemble to other guidewire components using conventional technology such as soldering or use of polymer adhesives due to inherent surface properties such as tenacious oxide layers. In one prior art guidewire, a nitinol wire is joined to the proximal end of a stainless steel wire by either a hypotube/glue arrangement, or dissimilar weld. This joint typically has an abrupt stiffens transition from the nitinol to the stainless steel, has complex manufacturing steps, and is expensive to manufacture.

In US 2005/027214 A1, it is described that a guide wire includes a distal end portion and a main body portion, wherein the main body portion includes a center layer formed of a first material, a surface layer formed of a second material, and an intermediate layer formed of a mixture of the first material and the second material.

In US 2018/311477 A1, there is described a guidewire made from a drawn filled tube composite wire. The composite wire has a stainless steel outer sheath jacketing a nitinol core wire. The drawn filled tube composite wire is ground at its distal end to expose the nitinol core, which has superelastic and kink resistant properties. The proximal end of the drawn filled tube is not ground or if ground, the outer sheath of stainless steel is not removed to an extent sufficient to expose the nitinol core.

The present invention allows for the design of a guidewire with a unitary core, rather than a core with proximal and distal segments joined together. Additionally, the core member of the present invention may be used with other wire designs to create guidewires with improved superelasticity and kink-resistance.

### SUMMARY OF THE INVENTION

It is an important aspect of the guidewire disclosed herein to utilize the structural features of a metal alloy having high yield strength for pushability and torque performance combined with a metal alloy having a high degree of elasticity for flexibility and trackability performance.

Various aspects and embodiments are set out in the appended claims.

In one example, useful for understanding the claims, an elongated tubular member has a proximal end and a distal end and includes an inner core member and an outer layer over the inner core member. The inner core member is formed from a first metal alloy and the outer layer is formed from a second metal alloy that is different than the first metal alloy. A feather edged joint defining a tapered transition segment is formed between the outer layer and the inner core member. Preferably a section of the outer layer is ground down to form the feather edged joint. The tapered transition segment extends from a first point on the outer layer where the nominal transverse wall thickness is constant, to a second point where the nominal transverse wall thickness of the outer layer is zero. In other words, the tapered segment extends between the first point where the wall thickness of the outer layer is thickest and then tapers (from the grinding process) to the second point where the wall thickness of the outer layer has been ground down to zero and the inner core member is exposed. In this embodiment, the inner core member is further ground at certain points along its length to form multiple tapered sections along a distal segment of the inner core member. Importantly, no portion of the outer layer surrounds the inner core member distal of the second point.

In one example, useful for understanding the claims, a drawn filled tube has a proximal end and a distal end and includes an inner core member and an outer layer over the inner core member. The inner core member is formed from a first metal alloy and the outer layer is formed from a second metal alloy that is different than the first metal alloy. A feather edged joint defining a tapered transition segment is formed between the outer layer and the inner core member. Preferably a section of the outer layer is ground down to form the feather edged joint. The tapered transition segment extends from a first point on the outer layer where the nominal transverse wall thickness is constant, to a second point where the nominal transverse wall thickness of the outer layer is zero. In other words, the tapered segment extends between the first point where the wall thickness of the outer layer is thickest and tapered (from the grinding process) to the second point where the wall thickness of the outer layer has been ground down to zero and the inner core member is exposed. In this embodiment, the inner core member is ground at certain points along its length to form multiple tapered sections along a distal segment of the inner core member. Importantly, no portion of the outer layer surrounds the inner core member distal of the second point.

In one example, useful for understanding the claims, the drawn filled tubing guidewire has a parabolic grind at a distal section thereof to take advantage of the nitinol inner core member.

In another example, useful for understanding the claims, a pressure sensor is attached to a sensor pocket formed in a proximal section of a drawn filled tubing guidewire.

### BRIEF DESCRIPTION OF THE DRAWINGS

FIG. 1 is a longitudinal cross-sectional view of a guidewire depicting an outer layer surrounding an inner core member.
FIG. 2 is a transverse cross-sectional view of the guidewire of FIG. 1 taken along line 2-2.
FIG. 3 is a longitudinal cross-sectional view of the guidewire of FIG. 1 depicting a feather edged joint.
FIG. 4 is a transverse cross-sectional view of the guidewire of FIG. 3 taken along line 4-4.
FIG. 5 is a transverse cross-sectional view of the guidewire of FIG. 3 taken along line 5-5.
FIG. 6 is a longitudinal cross-sectional view of the guidewire depicting the feather edged joint and multiple distal section tapers.
FIG. 7 is a graph depicting the bending stiffness of a drawn filled tubing guidewire of the invention compared to a commercially available guidewire.
FIG. 8 is a graph depicting the bending stiffness of a drawn filled tubing guidewire of the invention compared to a commercially available guidewire.
FIG. 9 is a graph depicting the bending stiffness constant diameter wires including of a drawn filled tubing guidewire of the invention compared to a commercially available guidewire.
FIG. 10 is an elevational view of a prior art guidewire depicting a distal section having multiple tapered sections.
FIG. 11 is an elevational view of a guidewire depicting a distal section having a parabolic grind profile.
FIG. 12 is a graph depicting the bending stiffness along the distal section of the guidewires shown in FIGS. 10 and 11.
FIG. 13. is a perspective view of a drawn filled tubing guidewire depicting a pressure sensor and sensor pocket.
FIG. 14 is a longitudinal cross-sectional view of the drawn filled tubing guidewire of FIG. 13.
FIG. 15 is a partial perspective view of the guidewire shown in FIG. 13 depicting the pressure sensor attached to the sensor pocket.
FIG. 16A is a longitudinal cross-sectional view of the guidewire shown in FIG. 15 depicting a polymer sleeve covering the power and data cable.
FIG. 16B is a transverse cross-sectional view taken along lines 16B-16B depicting the polymer sleeve covering the power and data cable.

### DETAILED DESCRIPTION OF THE PREFERRED EMBODIMENTS

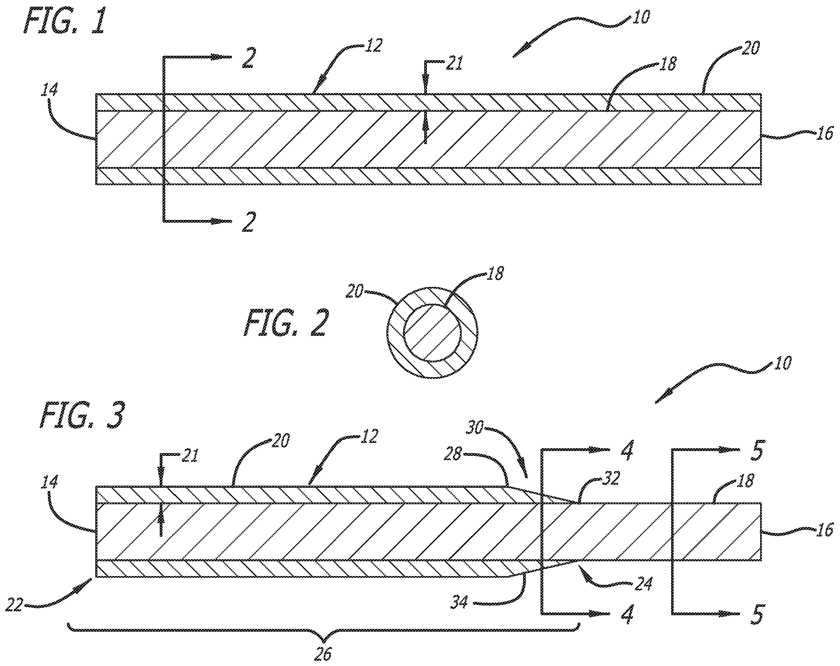

FIGS. 1-6 illustrate features of a guidewire 10 embodying features of the invention. A composite elongated tubular member 12 has a proximal end 14 and a distal end 16. The elongated tubular member has an inner core member 18 that extends from the proximal end 14 to the distal end 16 and preferably is a solid core wire formed from a linear elastic alloy or a superelastic alloy. An outer layer 20 covers or surrounds the inner core member 18 along elongated tubular member 12. A wall thickness 21 of the outer layer 20 is shown in FIGS. 1, 3 and 6 and is sometimes referred to herein as a single wall thickness 21. The outer layer 20 is formed from a precipitation hardenable material such as 35N LT, stainless steel, L605, MP35, MP35N, cobalt chromium, titanium, NiTiCo, NiTiCr, NiTi ternary alloys and similar metal alloys. More specifically, the outer layer 20 extends from the proximal end 14 in a distal direction to the distal end 16. It is preferred that the outer layer 20 tightly surrounds the inner core member 18 with no space or gaps between the structures. The inner core member 18 is preferably formed from linear elastic or superelastic alloys and can include NiTi, CuNiTi, NiTiCr, NiTiCo, and similar metal alloys. The elongated tubular member 12 is typically drawn filled tubing (DFT) that is commercially available from several sources such as Fort Wayne Metals, Fort Wayne, Indiana.

In one embodiment, shown in FIGS. 1-6, a portion of the outer layer 20 is ground down to expose the inner core member 18. The outer layer has a proximal end 22 and a distal end 24 and a proximal segment 26 that extends from the proximal end 22 to a first point 28 that is closer to the distal end 24 than the proximal end 22. In this embodiment, a feather edged joint 30 is formed by grinding down the outer layer 20 from the first point 28 to a second point 32. The outer layer 20 is ground down so that the feather edged joint 30 forms a tapered transition segment 34 that gradually tapers from the first point 28 to the second point 32. The tapered transition segment 34 of the feather edged joint 30 extends a length along the elongated tubular member 12 beginning at the first point 28 where the nominal transverse wall thickness of the outer layer 20 is a constant, and ends at the second point 32 where the nominal transverse wall thickness of the outer layer 20 is zero. The length of the tapered transition segment 34 extends from first point 28 to the second point 32 and is in the range of 0.079 inch (2.0 mm) to 1.000 inch (25.4 mm). Importantly, one critical aspect of the invention is to form a joint between two dissimilar metals such as 35N LT (the outer layer 20) and linear elastic or superelastic nitinol (the inner core member 18), which traditionally is difficult or impossible to achieve by welding, brazing, or the like. The feather edged joint 30 provides a seamless transition between the two metals thereby significantly improving the structural aspects of the joint as well as enhancing the tactile feel for the physician. In this embodiment, the inner core member 18 is further ground down to form multiple distal tapers to enhance flexibility in the distal portion of the guidewire. Starting at the second point 32 on the inner core member 18 and moving distally to a third point 36, the inner core member 18 has a first constant diameter section 40. Starting at the third point 36 and moving distally to a fourth point 42, the inner core member 18 is ground down to form a first tapered section 38. The inner core member 18 has a second constant diameter section 48 extending from the fourth point 42 to a fifth point 46. The inner core member 18 is ground down to form a second tapered section 44 extending from the fifth point 46 to a sixth point 50. The inner core member 18 has a third constant diameter section 52 that extends from the sixth point 52 to the distal end 16 of the elongated tubular member 12. Importantly, the nitinol distal portion of the guidewire having multiple tapers allows the guidewire to maneuver through tortuous anatomy without permanent deformation of the guidewire.

The overall length and diameter of guidewire 10 may be varied to suit the particular procedures in which it is to be used and is dependent on the materials from which it is constructed. Generally, the length of the guidewire 10 ranges from about 65 cm to about 350 cm, and more typically ranging from about 160 cm to about 200 cm. In one embodiment, the guidewire 10 is 180 cm long.

Commercially available guidewires for coronary and peripheral anatomy typically have lengths of about 175 cm or about 330 cm. Guidewire diameters generally range from about 0.008 inch to about 0.035 inch (0.2 to 0.9 mm), and more typically range from about 0.01 inch to about 0.018 inch (0.25 to 0.55 mm). Commercially available guide wires for coronary and peripheral use typically have diameters of 0.014 inch and 0.018 inch (0.036 mm and 0.46 mm, respectively).

As can be seen in Table 1, standard guidewire diameters are 0.014 inch, 0.018 inch, and 0.035 inch for most coronary and peripheral vessel applications. While other guidewire diameters are contemplated, these diameters comprise the vast majority of commercially available guidewires. Table 1 shows the ranges of diameters for a drawn filled tubing guidewire including nominal diameters and preferred diameters for the disclosed invention.

| **Table 1** (**inches**) | | | | | | | | |
|---|---|---|---|---|---|---|---|---|
| Standard Guidewire Diameters | Overall DFT Wire Diameters | | Preferred Overall DFT | | Nitinol Core Diameters | | Preferred Nitinol Core Diameters | |
| A | B | C | D | E | F | G | H | I |
| 0.014 | 0.013 | 0.014 | 0.0132 | 0.0135 | 0.0092 | 0.0128 | 0.0094 | 0.0125 |
| 0.018 | 0.0165 | 0.018 | 0.017 | 0.0175 | 0.0142 | 0.0148 | 0.0143 | 0.0145 |
| 0.035 | 0.029 | 0.035 | 0.028 | 0.032 | 0.025 | 0.03 | 0.026 | 0.029 |

| **Table 2 (inches)** | | | | | |
|---|---|---|---|---|---|
| Double Wall Thickness | | | | | |
| D-F | D-H | E-G | E-I | Wall Thickness | Wall Thickness |
| 0.004 | 0.0038 | 0.0007 | 0.001 | 0.0025 | 0.0012 |
| 0.0028 | 0.0027 | 0.0027 | 0.003 | 0.0029 | 0.0015 |
| 0.003 | 0.002 | 0.002 | 0.003 | 0.0035 | 0.0018 |

Referring to Table 1, a standard guidewire diameter for a DFT wire of 0.014 inch (column A) can range from 0.013 inch to 0.014 inch, as shown in columns B and C respectively. A preferred overall diameter DFT wire for the 0.014 inch wire (column A) can range from 0.0132 inch to 0.0135 inch, as shown in columns D and E respectively. The nominal nitinol core diameter range is shown in columns F and G, and the preferred nitinol core diameter range is shown in columns H and I. From Table 1, one can determine the overall diameter range for each of the three standard guidewires listed in column A, as well as the diameter ranges of the nitinol core wire.

The wall thickness of the outer layer 20 is derived from the dimensions set forth in Table 1. Referring to Table 2, by subtracting the preferred overall diameter of the DFT wire in column D from the nitinol core diameter in column F (0.0132 inch - 0.0092 inch = 0.004 inch), the wall thickness of the outer layer 20 is derived, as shown in column D-F. Since there are two wall thicknesses in the diameter dimension, the "average" wall thickness column in Table 2 is divided by two to obtain the wall thickness of the outer layer. For example, the preferred wall thickness for a 0.014 inch diameter guidewire is calculated by subtracting D (0.0132) from H (0.0094) to equal $\frac{0.0038 inch}{2}$ to equal 0.0019 inch wall thickness. The column headed "average" is calculated by the formula $\frac{\left(D-H\right) + \left(E-I\right)}{2}$, which is the total average wall thickness of the outer layer. The average wall thickness of a single layer of the outer layer is calculated in the column headed "average/2" and is calculated using the formula $\frac{\left(D-H\right) + \left(E-I\right)}{2} \div 2$. For example, in one embodiment, a 0.014 inch diameter DFT guidewire has an "average/2" outer layer single wall thickness 21 of 0.0012 inch $\frac{\left(D-H\right) - \left(E-I\right)}{2} \div 2$. In another embodiment, a 0.018 inch diameter DFT guidewire has an "average/2" outer layer single wall thickness 21 of 0.0015 inch. In another embodiment, a 0.035 inch diameter DFT guidewire has an "average/2" outer layer single wall thickness 21 of 0.0018 inch.

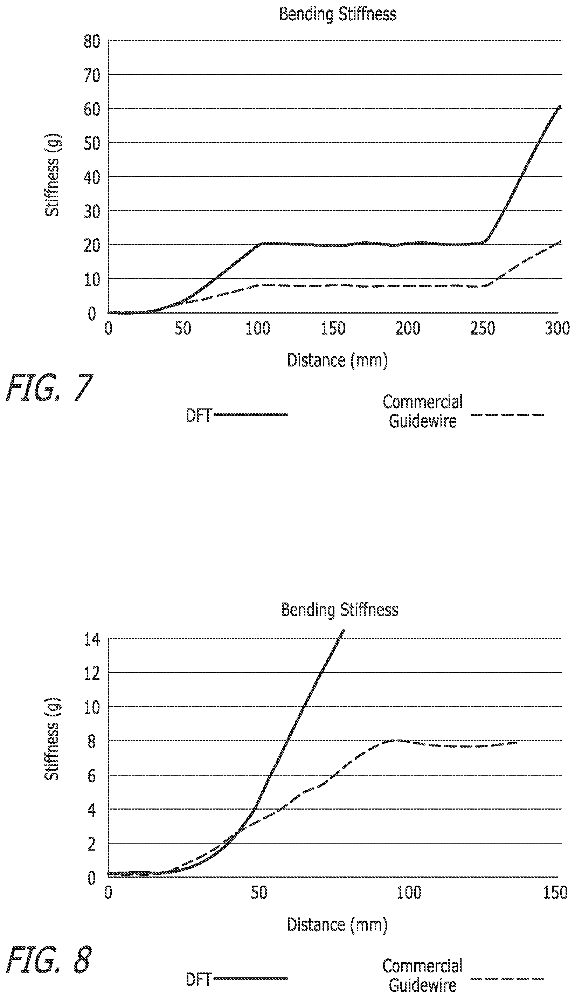

The guidewire 10 disclosed herein is formed from drawn filled tubing (DFT) and after processing as disclosed herein, including forming a feather edged joint 30, has bending stiffness characteristics similar or superior to commercially available guidewires. As shown in the graphs of FIGS. 7 and 8, the bending stiffness of a DFT guidewire 10 is compared to that of a commercially available guidewire manufactured and sold by Abbott Cardiovascular Systems, Inc., Santa Clara, California. The bending stiffness from the distal tip of each guidewire to about 50 mm is similar and shows a high degree of flexibility due to a very low bending stiffness. From about 50 mm to about 150 mm, the DFT guidewire 10 has a higher bending stiffness than the commercial guidewire, which means that DFT guidewire 10 has better pushability and torque response than the commercial guidewire.

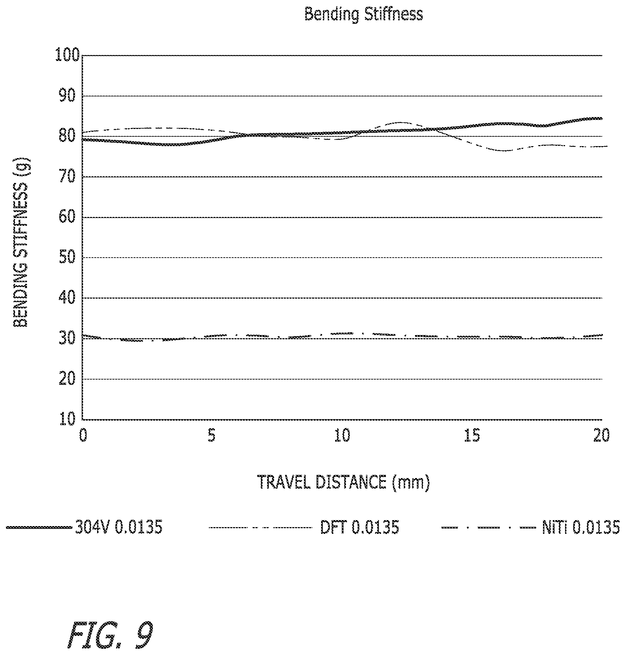

Referring to the graph in FIG. 9, the bending stiffness of a constant diameter guidewire of 0.0135 inch was compared among a superelastic nitinol guidewire, a 304V stainless steel guidewire, and a DFT guidewire. As can be seen in FIG. 9, the superelastic nitinol guidewire has a low and relatively constant bending stiffness. The DFT guidewire and the 304V stainless steel guidewire have similar high and relatively constant bending stiffness. Thus, comparing the graphs from FIGS. 7 and 8 to FIG. 9, it can be seen that once the DFT guidewire 10 has the distal portion of the outer layer removed and the multiple tapers formed in the distal portion, the DFT guidewire performs as well or better than the commercially available guidewires.

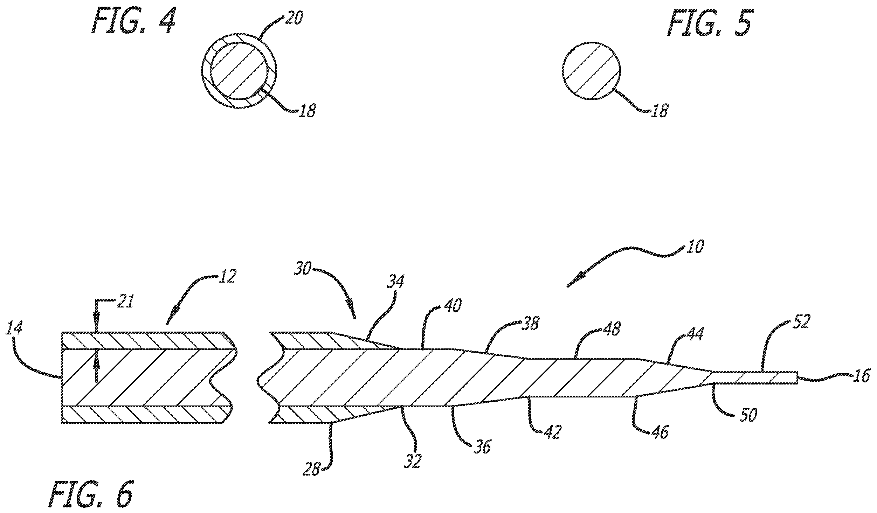

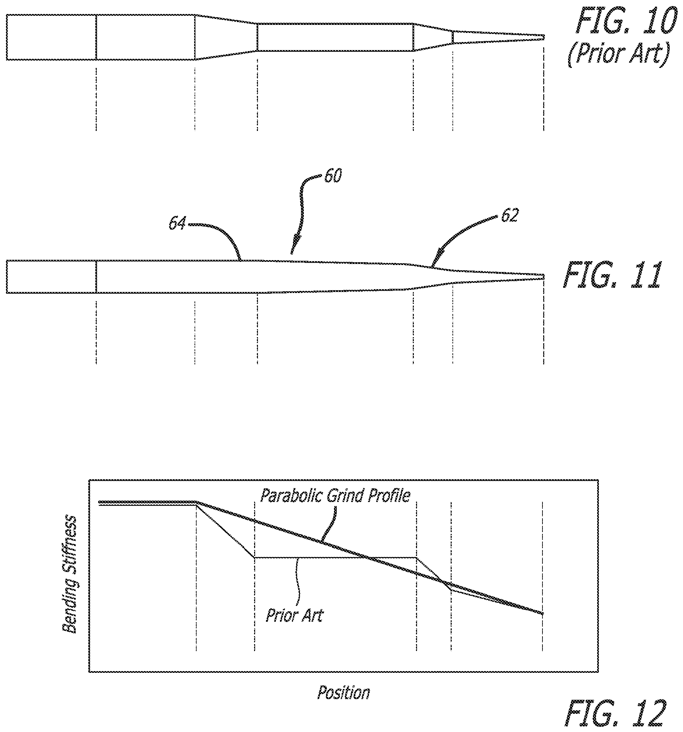

The bending stiffness of the drawn filled tubing (DFT) disclosed herein can also be altered by applying a parabolic grind along the nitinol distal section of the guidewire. A parabolic grind profile was described in commonly owned and commonly assigned U.S. Serial No. 16/671,044 filed October 31, 2019. Rather than grinding the nitinol inner core member 18 as shown in FIG. 6 with a first tapered section 38 and a second tapered section 44, a parabolic grind can be applied instead. Thus, referring to FIGS. 31-33 of U.S. Serial No. 16/671,044, now renumbered as FIGS. 10-12 herein, the distal section of the guidewire is reduced in cross-section to be more flexible when navigating tortuous vessels, such as coronary arteries. The distal section of the guidewire must be both flexible and pushable, that is the distal section must flex and be steerable through the tortuous arteries, and also have some stiffness so that it can be pushed or advanced through the arteries without bending or kinking. A prior art guidewire is shown in FIG. 10 and has a distal section comprised of tapered sections and core sections with no taper. The resulting bending stiffness is shown in the graph in FIG. 12 wherein the bending stiffness decreases at each tapered position, and the bending stiffness remains constant along the core section that is not tapered. The tapered distal section of the prior art guidewire of FIG. 10 provides abrupt changes in bending stiffness that can reduce the tactile feel to the physician when advancing the guidewire through tortuous anatomy. In fact, in some prior art guidewires, the abrupt change in bending stiffness can result in the distal tip of the guidewire to kink or prolapse into a side branch vessel. Prolapse can be dangerous to the patient in that the artery can be damaged or punctured. Importantly, it is preferred to maintain the outer diameter of the core section as far distal as possible to maintain torque. Each tapered section loses torque, which is critical in advancing the guidewire through tortuous vessels.

In keeping with the invention, a parabolic distal section 62 of a guidewire 60 is shown in FIG. 11 wherein a significant portion of the distal section has been ground to form a continuous taper. More specifically, the continuous taper is formed by a parabolic grind along parabolic distal section 62 of the guidewire 60. The parabolic grind provides a smooth curvilinear transition along section 62 that is highly flexible and yet maintains a linear change in stiffness as shown in the graph of FIG. 12. Not only is parabolic distal section 62 flexible, but it has a linear change in stiffness thereby providing excellent torque and tactile feedback to the physician when advancing the guidewire through tortuous anatomy. A tapered section 64 that is not curvilinear (not a parabolic grind section) is located on the guidewire 60 distal of the parabolic distal section 62 and it provides reduced bending stiffness and a linear change in bending stiffness as shown in the graph of FIG. 12. It is intended that the parabolic grind profile be applied to only the nitinol inner core member along the parabolic distal section 62.

Bending stiffness can be measured in a variety of ways. Typical methods of measuring bending stiffness include extending a portion of the sample to be tested from a fixed block with the sample immovably secured to the fixed block and measuring the amount of force necessary to deflect the end of the sample that is away from the fixed block a predetermined distance. A similar approach can be used by fixing two points along the length of a sample and measuring the force required to deflect the middle of the sample a fixed amount. Those skilled in the art will realize that a large number of variations on these basic methods exist including measuring the amount of deflection that results from a fixed amount of force on the free end of a sample, and the like. Other methods of measuring bending stiffness may produce values in different units of different overall magnitude, however, it is believed that the overall shape of the graph will remain the same regardless of the method used to measure bending stiffness.

The embodiments of the drawn filled tubing (DFT) guidewire disclosed herein are particularly suitable for use in conjunction with a pressure sensor. During some medical interventions, it may be desirable to measure and/or monitor the blood pressure within a blood vessel. For example, some medical devices may include pressure sensors that allow a clinician to monitor blood pressure. Such devices may be useful in determining fractional flow reserve (FFR), which may be understood as the pressure after a stenosis relative to the pressure before the stenosis. A number of pressure sensing devices, however, may pose technical challenges for steering, tracking, torqueing or otherwise navigating the device within the vasculature. For example, medical devices may include a relatively stiff pressure sensor located at or near the distal tip of the device and/or a sensor housing (in which the sensor is mounted) that may also be relatively stiff. Disclosed herein are a number of medical device that include pressure sensing capabilities and may be more easily steered, tracked, torqued, and/or otherwise navigated through the anatomy. The DFT guidewire of the present invention is used to carry a pressure sensor through the coronary and peripheral vessels to measure blood pressure and possibly FFR, as well as other measurements.

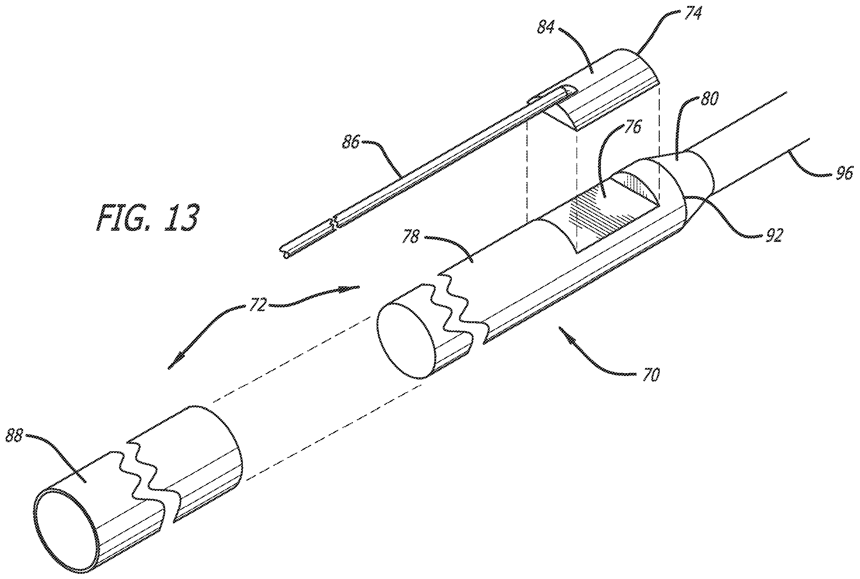

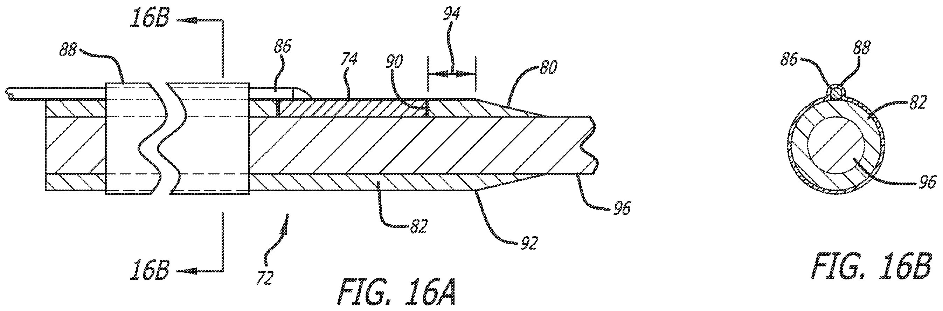

In one embodiment, shown in FIGS. 13-16B, a drawn filled tubing (DFT) guidewire 70 is processed according to the embodiment shown in FIGS. 1-6. The difference, however, is in this embodiment the proximal section 72 is adapted to carry a pressure sensor 74 (or any other type of measuring device). The pressure sensor 74 can be any sensor known in the art and may include a semiconductor (e.g., silicon wafer) pressure sensor, piezoelectric pressure sensor, a fiber optic or optical pressure sensor, a Fabry-Perot type sensor, an ultrasound transducer and/or ultrasound pressure sensor, a magnetic pressure sensor, a solid-state pressure sensor, and the like. In this embodiment, a sensor pocket 76 is formed in the proximal section 72 of the drawn filled tubing 70 for mounting the pressure sensor 74. The sensor pocket 76 can be formed by grinding the outer surface 78 of DFT guidewire 70 to a depth and shape that is configured to receive the pressure sensor 74 without compromising the integrity of the DFT guidewire 70. Also, it is preferred that the pressure sensor 74 be configured so as to not disrupt blood flow or impede the movement of the DFT guidewire 70 through the vasculature. Preferably, the sensor pocket 76 is positioned on the DFT guidewire 70 just proximal of the tapered transition segment 80 where the outer layer 82 is first ground down to form the tapered transition segment 80. The grinding procedure for forming the sensor pocket 76 can be performed by any method well known in the prior art.

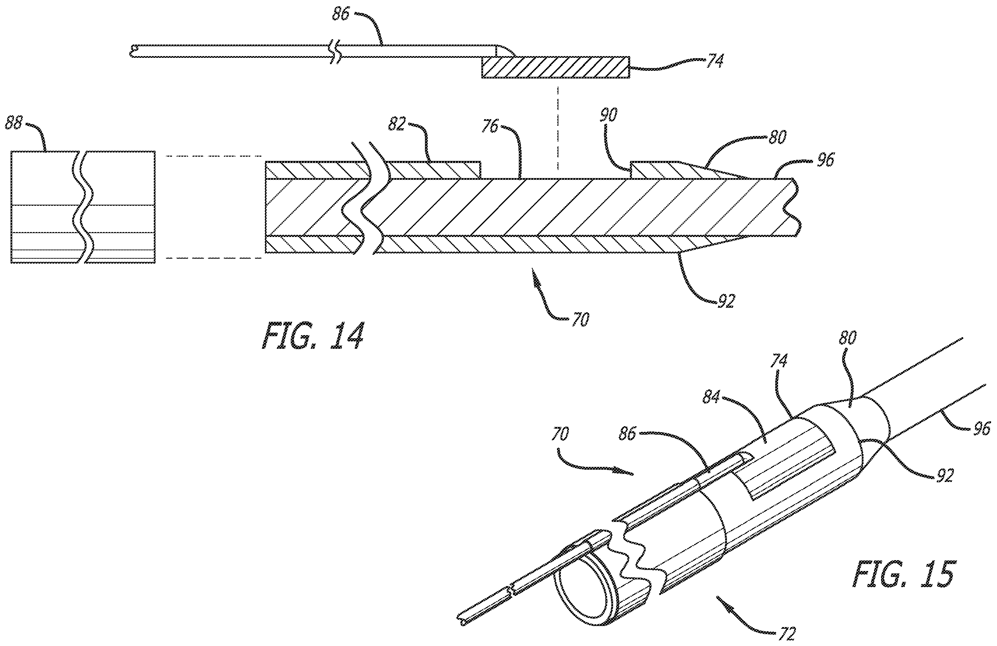

Pressure sensors 74 are well known in the art and can take many different sizes and shapes. As shown in FIG. 15, for example, the pressure sensor 74 has a contoured outer surface 84 to conform to the curved outer surface 78 of the DFT guidewire 70 so that there are no sharp edges that could cause injury to the vasculature. The pressure sensor 74 can be attached to the sensor pocket 76 by any known means including adhesive, laser welding, soldering, brazing, and the like.

In further reference to FIGS. 13-16B, a power and data transmission cable 86 is attached to the pressure sensor 74 and the cable 86 extends proximally along the outer surface 78 of the DFT guidewire 70 to a location outside of the patient so that it can be attached to the appropriate monitoring equipment. A polymer sleeve 88 is formed over the cable 86 and at least a portion of the outer surface 78 of the DFT guidewire 70 in order to protect the cable 86 and prevent it from interfering with the movement of the guidewire in the vasculature. While a polymer sleeve 88 is preferred, other covers are contemplated such as polymer bands, radiopaque marker bands, braided materials, and the like.

It is preferred that the sensor pocket 76 and the attached pressure sensor 74 be positioned very close to the tapered transition segment 80, but proximal thereto. In one embodiment, a distal edge 90 of the sensor pocket 76 is a length 94 between 0.1 mm and 30.0 mm from a first point 92 which marks the beginning of the tapered transition segment 80. In another embodiment, the length 94 is between 0.1 mm and 10.0 mm.

Referring to FIG. 14, the depth of the sensor pocket 76 is shown to be equal to the thickness of the outer layer 82. Depending on the thickness of the pressure sensor 74, the depth of the sensor pocket 76 can be more or less than depicted in FIG. 14, and extends into an inner core member 96 which is formed from nitinol as previously described. It is preferred that the depth of the sensor pocket 76 into the inner core member be minimal, however, it is possible that the sensor pocket 76 extends half way through the diameter of the DFT guidewire 70, which means the pocket would extend well into the nitinol inner core member 96.

Guidewires like the ones disclosed herein typically will have one or more coils attached to the distal end as well as a solder structure, neither of which are shown. Further, the drawn filled tubing is manufactured by know means and need not be further described. The multiple distal tapers are formed by centerless grinding machines and the method of grinding is well known and need not be further described.

With respect to the embodiment shown in FIGS. 13-16B, the DFT guidewire 70 has an inner core member 96 extending the entire length of the guidewire 70. In some embodiments, it is preferred that the inner core member 96 extend only in a distal portion of the guidewire so that the outer layer 82 covers only a short length of the proximal end of the inner core member 96. In one embodiment, the outer layer 82 overlaps and covers less than 2.5 cm of the proximal end of the inner core member 96.

While the present invention has been described and illustrated in terms of its use as an intravascular guidewire, it will be apparent to those skilled in the art that the present invention can be applied to other medical devices. Moreover, modifications and improvements may be made to the above-described exemplary embodiments without departing from the scope of the invention.

## Claims

1. A guidewire (10), comprising:
an elongated tubular member (12) having a proximal end (14) and distal end (16);
the elongated tubular member having an inner core member (18) formed from a first material and an outer layer (20) formed from a second material surrounding at least a portion of the inner core member; and
a feather edged joint (30) formed from a section of the outer layer defining a tapered transition segment (34) between the outer layer and the inner core member, wherein the tapered transition segment is formed from the second material and has a length in a range of 2.0 mm (0.079 inch) to 25.4 mm (1.000 inch).

2. The guidewire of claim 1, wherein the first material is a first metal alloy that is different from a second metal alloy of the second material.

3. The guidewire of claim 2, wherein the first metal alloy is formed from a linear elastic alloy or a superelastic alloy, optionally wherein the linear elastic alloy of the first metal alloy is taken from the group of metal alloys including NiTi, CuNiTi and NiTiCr.

4. The guidewire of claim 3, wherein the second metal alloy is taken from the group of metal alloys including 35N LT, stainless steel, L605, MP35, MP35N, cobalt-chromium, titanium, NiTiCo, NiTiCr, and NiTi ternary alloys.

5. The guidewire of claim 4, wherein the inner core member is a solid wire, optionally wherein the inner core member has a nominal diameter at an untapered section in the range of 0.24mm to 0.74mm (0.0094 inch to 0.029 inch), further optionally wherein the outer layer has a nominal transverse single wall thickness at an untapered section in the range of 0.030mm to 0.046mm (0.0012 inch to 0.0018 inch).

6. The guidewire of claim 1, wherein the tapered transition segment of the feather edged joint extends a length along the elongated tubular member beginning at a first point (28) where the nominal transverse single wall thickness of the outer layer is a constant, and ends at a second point (32) where the nominal transverse single wall thickness of the outer layer is zero.

7. The guidewire of claim 6, wherein the outer layer tapers distally from the first point to the second point to form the feather edged joint.

8. The guidewire of claim 7, wherein the inner core member has a first constant diameter section (40) extending distally from the second point to a third point (36),
further optionally wherein the inner core member has a first tapered section (38) extending from the third point to a fourth point (42),
further optionally wherein the inner core member has a second constant diameter section (48) extending from the fourth point to a fifth point (46),
further optionally wherein the inner core member has a second tapered section (44) extending from the fifth point to a sixth point (50),
further optionally wherein the inner core member has a third constant diameter section (52) extending from the sixth point to the distal end of the elongated member.

9. The guidewire of claim 8, wherein the third constant diameter section has a nominal diameter in the range from 0.05mm to 0.15mm (0.002 inch to 0.0060 inch), and the third constant diameter section is less than the first constant diameter section and the second constant diameter section, optionally wherein no portion of the outer layer surrounds the inner core member from the second point to the distal end of the elongated tubular member.

10. The guidewire of claim 1, wherein either:
the inner core member has a preferred diameter at an untapered section, for a 0.36mm (0.014 inch) guidewire, in the range from 0.24mm to 0.318mm (0.0094 inch to 0.0125 inch); or
the inner core member has a preferred diameter at an untapered section, for a 0.46mm (0.018 inch) guidewire, in the range from 0.363mm to 0.368mm (0.0143 inch to 0.0145 inch); or
the inner core member has a preferred diameter at an untapered section, for a 0.89mm (0.035 inch) guidewire, in the range from 0.660mm to 0.737mm (0.0260 inch to 0.0290 inch).

11. The guidewire of any preceding claim, wherein the elongated tubular member is an elongated drawn filled tube.

12. The guidewire of claim 1, wherein the inner core member has a constant diameter section (40) and a tapered distal section, and the tapered transition segment extends along the constant diameter section and ends at a point proximal to the tapered distal section of the inner core member.

13. The guidewire of claim 1, wherein the outer layer tightly surrounds the inner core member with no space or gaps between the structures, optionally wherein the outer layer and the inner core member are formed of different metal alloy materials.

## Patentansprüche

1. Führungsdraht (10), umfassend:
ein langgestrecktes, rohrförmiges Element (12) mit einem proximalen Ende (14) und einem distalen Ende (16);
wobei das langgestreckte, rohrförmige Element ein aus einem ersten Material gebildetes inneres Kernelement (18) und eine aus einem zweiten Material gebildete äußere Schicht (20) aufweist, die zumindest einen Abschnitt des inneren Kernelements umgibt; sowie
eine aus einem Abschnitt der äußeren Schicht gebildete, abgeflachte Verbindungsstelle (30), die ein verjüngtes Übergangssegment (34) zwischen der Außenschicht und dem inneren Kernelement definiert, wobei das verjüngte Übergangssegment aus dem zweiten Material gebildet ist und eine Länge im Bereich von 2,0 mm (0,079 Zoll) bis 25,4 mm (1,000 Zoll) aufweist.

2. Führungsdraht nach Anspruch 1, bei dem das erste Material eine erste Metalllegierung ist, die sich von einer zweiten Metalllegierung des zweiten Materials unterscheidet.

3. Führungsdraht nach Anspruch 2, bei dem die erste Metalllegierung aus einer linear-elastischen Legierung oder einer superelastischen Legierung gebildet ist, wobei optional die linear-elastische Legierung der ersten Metalllegierung aus der Gruppe von Metalllegierungen ausgewählt ist, die NiTi, CuNiTi und NiTiCr umfasst.

4. Führungsdraht nach Anspruch 3, bei dem die zweite Metalllegierung aus der Gruppe von Metalllegierungen ausgewählt ist, die 35N LT, Edelstahl, L605, MP35, MP35N, Kobalt-Chrom, Titan, NiTiCo, NiTiCr und ternäre NiTi-Legierungen umfasst.

5. Führungsdraht nach Anspruch 4, bei dem das innere Kernelement ein massiver Draht ist, wobei optional das innere Kernelement einen Nenndurchmesser an einem nicht verjüngten Abschnitt im Bereich von 0,24 mm bis 0,74 mm (0,0094 Zoll bis 0,029 Zoll) aufweist, wobei ferner optional die äußere Schicht an einem nicht verjüngten Abschnitt eine nominale Einzelwandstärke in Querrichtung im Bereich von 0,030 mm bis 0,046 mm (0,0012 Zoll bis 0,0018 Zoll) aufweist.

6. Führungsdraht nach Anspruch 1, bei dem sich das verjüngte Übergangssegment der abgeflachten Verbindungsstelle über eine Länge entlang des langgestreckten, rohrförmigen Elements erstreckt, beginnend an einem ersten Punkt (28), an dem die nominale Einzelwandstärke in Querrichtung der äußeren Schicht konstant ist, und endend an einem zweiten Punkt (32), an dem die nominale Einzelwandstärke in Querrichtung der äußeren Schicht null ist.

7. Führungsdraht nach Anspruch 6, bei dem sich die äußere Schicht vom ersten Punkt zum zweiten Punkt in distaler Richtung verjüngt, um die abgeflachte Verbindungsstelle zu bilden.

8. Führungsdraht nach Anspruch 7, bei dem das innere Kernelement einen ersten Abschnitt (40) mit konstantem Durchmesser aufweist, der sich distal vom zweiten Punkt zu einem dritten Punkt (36) erstreckt,
bei dem ferner optional das innere Kernelement einen ersten verjüngten Abschnitt (38) aufweist, der sich vom dritten Punkt zu einem vierten Punkt (42) erstreckt,
bei dem ferner optional das innere Kernelement einen zweiten Abschnitt (48) mit konstantem Durchmesser aufweist, der sich vom vierten Punkt bis zu einem fünften Punkt (46) erstreckt,
bei dem ferner optional das innere Kernelement einen zweiten verjüngten Abschnitt (44) aufweist, der sich vom fünften Punkt bis zu einem sechsten Punkt (50) erstreckt,
bei dem ferner optional das innere Kernelement einen dritten Abschnitt mit konstantem Durchmesser (52) aufweist, der sich vom sechsten Punkt bis zum distalen Ende des langgestreckten Elements erstreckt.

9. Führungsdraht nach Anspruch 8, bei dem der dritte Abschnitt mit konstantem Durchmesser einen Nenndurchmesser im Bereich von 0,05 mm bis 0,15 mm (0,002 Zoll bis 0,0060 Zoll) aufweist, und der dritte Abschnitt mit konstantem Durchmesser kleiner ist als der erste Abschnitt mit konstantem Durchmesser und der zweite Abschnitt mit konstantem Durchmesser, wobei optional kein Abschnitt der äußeren Schicht das innere Kernelement vom zweiten Punkt bis zum distalen Ende des langgestreckten, rohrförmigen Elements umgibt.

10. Führungsdraht nach Anspruch 1, bei dem entweder:
das innere Kernelement an einem nicht verjüngten Abschnitt eines 0,36 mm (0,014 Zoll) starken Führungsdrahts einen bevorzugten Durchmesser aufweist, der im Bereich von 0,24 mm bis 0,318 mm (0,0094 Zoll bis 0,0125 Zoll) liegt; oder
das innere Kernelement an einem nicht verjüngten Abschnitt eines 0,46 mm (0,018 Zoll) starken Führungsdrahts einen bevorzugten Durchmesser im Bereich von 0,363 mm bis 0,368 mm (0,0143 Zoll bis 0,0145 Zoll) aufweist; oder
das innere Kernelement an einem nicht verjüngten Abschnitt eines 0,89 mm (0,035 Zoll) starken Führungsdrahts einen bevorzugten Durchmesser im Bereich von 0,660 mm bis 0,737 mm (0,0260 Zoll bis 0,0290 Zoll) aufweist.

11. Führungsdraht nach einem der vorstehenden Ansprüche, bei dem das langgestreckte, rohrförmige Element ein langgestrecktes, gezogenes, ausgefülltes Rohr ist.

12. Führungsdraht nach Anspruch 1, bei das innere Kernelement einen Abschnitt mit konstantem Durchmesser (40) und einen verjüngten distalen Abschnitt aufweist und sich das verjüngte Übergangssegment entlang des Abschnitts mit konstantem Durchmesser erstreckt und an einem Punkt endet, der proximal zum verjüngten distalen Abschnitt des inneren Kernelements liegt.

13. Führungsdraht nach Anspruch 1, bei dem die äußere Schicht das innere Kernelement dicht umgibt, ohne dass zwischen den Strukturen Zwischenräume oder Lücken bestehen, wobei die äußere Schicht und das innere Kernelement optional aus unterschiedlichen Metalllegierungen bestehen.

## Revendications

1. Fil-guide (10), comprenant :
un élément tubulaire allongé (12) présentant une extrémité proximale (14) et une extrémité distale (16) ;
l'élément tubulaire allongé présentant un élément de noyau interne (18) formé à partir d'un premier matériau et une couche externe (20) formée à partir d'un second matériau entourant au moins une partie de l'élément de noyau interne ; et
un raccord à bord aminci (30) formée à partir d'une section de la couche externe définissant un segment de transition effilé (34) entre la couche externe et l'élément de noyau interne, dans lequel le segment de transition effilé est formé à partir du second matériau et présente une longueur dans une plage de 2,0 mm (0,079 pouce) à 25,4 mm (1 000 pouces) .

2. Fil-guide selon la revendication 1, dans lequel le premier matériau est un premier alliage métallique qui est différent d'un second alliage métallique du second matériau.

3. Fil-guide selon la revendication 2, dans lequel le premier alliage métallique est formé à partir d'un alliage élastique linéaire ou d'un alliage super-élastique, facultativement dans lequel l'alliage élastique linéaire du premier alliage métallique est pris dans le groupe d'alliages métalliques incluant NiTi, CuNiTi et NiTiCr.

4. Fil-guide selon la revendication 3, dans lequel le second alliage métallique est pris dans le groupe d'alliages métalliques incluant les alliages ternaires 35N LT, acier inoxydable, L605, MP35, MP35N, cobalt-chrome, titane, NiTiCo, NiTiCr et NiTi.

5. Fil-guide selon la revendication 4, dans lequel l'élément de noyau interne est un fil solide, facultativement dans lequel l'élément de noyau interne présente un diamètre nominal au niveau d'une section non effilée dans la plage de 0,24 mm à 0,74 mm (0,0094 pouce à 0,029 pouce), en outre facultativement dans lequel la couche externe présente une épaisseur de paroi unique transversale nominale au niveau d'une section non effilée dans la plage de 0,030 mm à 0,046 mm (0,0012 pouce à 0,0018 pouce).

6. Fil-guide selon la revendication 1, dans lequel le segment de transition effilé du raccord à bord aminci s'étend sur une longueur le long de l'élément tubulaire allongé en commençant en un premier point (28) où l'épaisseur de paroi unique transversale nominale de la couche externe est une constante, et se termine en un deuxième point (32) où l'épaisseur de paroi unique transversale nominale de la couche externe est nulle.

7. Fil-guide selon la revendication 6, dans lequel la couche externe est effilée distalement du premier point au deuxième point pour former le raccord à bord aminci.

8. Fil-guide selon la revendication 7, dans lequel l'élément de noyau interne présente une première section de diamètre constant (40) s'étendant de manière distale du deuxième point à un troisième point (36),
en outre facultativement dans lequel l'élément de noyau interne présente une première section conique (38) s'étendant du troisième point à un quatrième point (42),
en outre, facultativement, dans lequel l'élément de noyau interne présente une deuxième section de diamètre constant (48) s'étendant du quatrième point à un cinquième point (46),
en outre, facultativement, dans lequel l'élément de noyau interne présente une deuxième section effilée (44) s'étendant du cinquième point à un sixième point (50),
en outre facultativement dans lequel l'élément de noyau interne présente une troisième section de diamètre constant (52) s'étendant du sixième point à l'extrémité distale de l'élément allongé.

9. Fil-guide selon la revendication 8, dans lequel la troisième section de diamètre constant présente un diamètre nominal dans la plage de 0,05 mm à 0,15 mm (0,002 pouce à 0,0060 pouce), et la troisième section de diamètre constant est inférieure à la première section de diamètre constant et à la deuxième section de diamètre constant, facultativement dans lequel aucune partie de la couche externe n'entoure l'élément de noyau interne à partir du deuxième point vers l'extrémité distale de l'élément tubulaire allongé.

10. Fil-guide selon la revendication 1, dans lequel soit :
l'élément de noyau interne présente un diamètre préféré au niveau d'une section non effilée, pour un fil-guide de 0,36 mm (0,014 pouce), dans la plage de 0,24 mm à 0,318 mm (0,0094 pouce à 0,0125 pouce) ; ou
l'élément de noyau interne présente un diamètre préféré au niveau d'une section non effilée, pour un fil-guide de 0,46 mm (0,018 pouce), dans la plage de 0,363 mm à 0,368 mm (0,0143 pouce à 0,0145 pouce) ; ou
l'élément de noyau interne présente un diamètre préféré au niveau d'une section non effilée, pour un fil-guide de 0,89 mm (0,035 pouce), dans la plage de 0,660 mm à 0,737 mm (0,0260 pouce à 0,0290 pouce).

11. Fil-guide selon l'une quelconque des revendications précédentes, dans lequel l'élément tubulaire allongé est un tube rempli étiré allongé.

12. Fil-guide selon la revendication 1, dans lequel l'élément de noyau interne présente une section de diamètre constant (40) et une section distale effilée, et le segment de transition effilé s'étend le long de la section de diamètre constant et se termine en un point proximal à la section distale effilée de l'élément de noyau interne.

13. Fil-guide selon la revendication 1, dans lequel la couche externe entoure étroitement l'élément de noyau interne sans espace ni jeu entre les structures, facultativement dans lequel la couche externe et l'élément de noyau interne sont formés de différents matériaux d'alliage métallique.
